# EUROPEAN PATENT SPECIFICATION

(11) **EP 0 706 511 B1**
(45) Date of publication and mention of the grant of the patent: **25.08.1999**
(21) Application number: 94920720.3
(22) Date of filing: 22.06.1994
(51) Int. Cl.: C07C 303/00, C07C 309/12, C07C 303/32

(54) **PREPARATION OF AMMONIUM HYDROXYALKYL/ALKANOYLALKYL SULFONATES**
HERSTELLUNG VON AMMONIUM-HYDROXYALKYL/ALKANOYLALKYL-SULFONATEN
PROCEDE POUR PREPARER DES HYDROXYALKYL/ALCANOYLALKYL SULFONATES D'AMMONIUM

(30) Priority: 29.06.1993 US 85062; 29.06.1993 US 85061
(43) Date of publication of application: 17.04.1996
(73) Proprietor: BASF CORPORATION, Mount Olive, New Jersey 07828-1234 (US)
(72) Inventor: BRIODY, Robert, G., Apollo, PA 15613 (US); DOTY, Amy, E., Crystal Lake, IL 60014 (US); GOVINDAN, Cheruthur, Pittsburgh, PA 15239 (US); NEHMSMANN, Louis, J., Apollo, PA 15613 (US); WANG, Alan, E., Hoffman Estates, IL 60195 (US)
(74) Representative: Sternagel, Hans-Günther, Dr.
(86) International application number: US9406601
(87) International publication number: WO9501331

(56) References cited:
- CA-A- 687 540
- DE-B- 1 058 500
- GB-A- 869 744
- GB-A- 917 952
- US-A- 2 820 818

## Description

The present invention relates to the preparation of ammonium hydroxyalkyl sulfonates, e.g. ammonium isethionate, which are particularly useful in the production of ammonium alkanoyl alkyl sulfonate surface-active materials, and more particularly relates to the preparation of ammonium salts of fatty acid esters of hydroxyalkyl sulfonic acids. Still more particularly, the present invention relates to a process for utilizing ammonium isethionate in solution for preparing surface-active materials of the general formula RCO(O)R'SO₃M, wherein R is a monovalent aliphatic hydrocarbon radical having from 5 to 23 carbon atoms, R' is a bivalent alkylene radical containing from 2 to 4 carbon atoms and M is the ammonium cation. In a preferred embodiment, the present invention relates to the use of ammonium isethionate in aqueous solution for the preparation of ammonium salts of alkanoyl isethionates, e.g. ammonium cocoyl isethionate. In addition, the present invention relates to preventing corrosion of the reaction vessel when such reactions are carried out in metal reaction vessels, e.g., stainless steel autoclaves, which without proper preventative steps results in corrosion of the vessel along with metallic contamination, for example, iron, and discoloration of the product.

Alkali metal, i.e. sodium or potassium, salts of alkanoyl alkyl sulfonates are described in U.S. Patent 3,429,136. Of such materials, sodium cocoyl isethionate is the most widely used commercially in personal care applications, e.g. in synthetic detergent bars, as described in U.S. Patent 4,663,070. Sodium cocoyl isethionate (SCI) is practically insoluble in water at room temperature, and hence its use in clear liquid formulations such as shampoos is limited.

In contrast, copending U.S. Serial No. 07/721,741, filed June 26, 1991, discloses that the ammonium salt of cocoyl isethionic acid, i.e. ammonium cocoyl isethionate (ACI), is very soluble in water at room temperature. Because of its high solubility in water, it has been found that clear, i.e. non-turbid, solutions of up to about 40 weight percent of relatively pure ACI, i.e. ACI of at least 85 percent anionic activity, may be prepared. At present, there are no known commercial sources for ammonium alkanoyl alkyl sulfonates, e.g. ammonium cocoyl isethionate. While U.S. Patent No. 4,663,070 refers generally to ammonium salts of C₁₀ to C₁₆ acyl isethionates, a process for preparing such materials, particularly of high purity and anionic activity, has not been described, particularly with respect to ammonium isethionate in aqueous solution.

In U.S. Serial No. 07/721,741, it is disclosed that ammonium alkanoyl alkyl sulfonates, e.g. ammonium alkanoyl isethionates such as ammonium cocoyl isethionate, may be prepared by heating an ammonium hydroxyalkyl sulfonate, e.g. ammonium isethionate, with a fatty acid having from about 6 to about 24 carbon atoms at temperatures below which charring of the ammonium alkanoyl alkyl sulfonate product occurs. It is further disclosed therein that the aforedescribed solid product may be removed from the reaction vessel as an aqueous solution without serious decomposition due to hydrolysis by mixing a small amount of an alkaline reagent, preferably a high boiling, relatively non-volatile, relatively water-free organic amine, e.g. a tertiary amine such as triethanolamine, with the product to adjust its acidity to a near neutral pH of from about 6 to about 8, and subsequently dissolving the product in water, thereby to form an aqueous solution of the ammonium alkanoyl alkyl sulfonate. Substantially clear, i.e. non-turbid, solutions of such a product may be used in cosmetic applications such as clear shampoo formulations.

U.S. Patent 2,820,818 discloses a process for the preparation of salts of hydroxy aliphatic sulfonic acids, which process comprises the steps of adding sulfur dioxide to an aqueous solution of an alkali until the pH value of the solution, measured at room temperature, i.e. at about 25°C, is between 4.5 and about 8, thereafter adding an epoxy aliphatic compound and a further amount of sulfur dioxide to the aqueous solution under reaction conditions and in such a manner as to maintain the pH of the solution between 4.5 and about 8 until substantially one molecular proportion of sulfur dioxide has been added per molecular proportion of alkali originally charged to the solution, and continuing the addition of the epoxy aliphatic compound until the alkali salt of the sulfuruous acid has been substantially consumed in the reaction mixture.

### SUMMARY OF THE INVENTION

The present invention relates to a method for producing ammonium alkanoyloxy alkyl sulfonate of the general formula I

RCO(O)R'SO₃M (I)

wherein R is a monovalent hydrocarbon residue of a fatty acid containing from 6 to 24 carbon atoms, R' is a bivalent hydrocarbon radical containing from 2 to 4 carbon atoms, and M is the ammonium cation. The invention comprises:
(a) reacting an aqueous solution of ammonium bisulfite with an alkylene oxide containing from 2 to 4 carbon atoms while maintaining the solids concentration of the reaction mixture at less than 70 weight percent ind the pH of the reaction mixture in the range of 4.3 to 7 until the reaction is at least 90 percent complete, as measured by the amount of residual sulfite in the reaction mixture, thereby to produce a product consisting essentially of ammonium hydroxyalkyl sulfonate of the general formula II

   HOR'SO₃ M (II)

   wherein R' and M are as defined above, said product containing alkylene glycol by-product;
(b) stripping ammonium hydroxyalkyl sulfonate prepared in (a) to remove alkylene glycol; and
(c) reacting ammonium hydroxyalkyl sulfonate having not more than 1 weight percent alkylene glycol, not more than 2 weight percent alkanolamine, and not more than about 2 weight percent ammonium sulfate, all based on the weight of the ammonium hydroxyalkyl sulfonate, with a fatty acid containing from 6 to 24 carbon atoms at a temperature in the range of from 150 to 200°C, thereby to produce an ammonium alkanoyloxy alkyl sulfonate of the above general formula, a 25 to 35 weight percent aqueous solution of which is non-turbid at room temperature.

The invention further relates to a clear liquid detergent composition comprising an aqueous solution of the above ammonium alkanoyloxy alkyl sulfonate of the general formula I.

### SUMMARY OF THE INVENTION

The present invention relates to ammonium alkanoyl alkyl sulfonates, e.g. ammonium cocoyl isethionate, prepared from a fatty acid, e.g. coco fatty acid, and an ammonium hydroxyalkyl sulfonate, e.g. ammonium isethionate, wherein the ammonium hydroxyalkyl sulfonate, e.g. ammonium isethionate, is prepared by reacting ammonium bisulfite with an alkylene oxide, e.g. ethylene oxide, under controlled pH conditions. The method of the present invention yields substantially pure ammonium hydroxyalkyl sulfonate, e.g. ammonium isethionate, having low levels of impurities such as ammonium sulfate, alkanolamine, e.g. ethanolamine, and alkylene glycol, e.g. ethylene glycol. As a result of this method of preparing the ammonium hydroxyalkyl sulfonate, e.g. ammonium isethionate, the ammonium hydroxyalkyl sulfonate may be used directly as an aqueous solution to react with a fatty acid, e.g. coco fatty acid, to form ammonium alkanoyl alkyl sulfonate, e.g. ammonium cocoyl isethionate. Consequently, the present method avoids the need to purify the ammonium hydroxyalkyl sulfonate, e.g. ammonium isethionate, by crystallization in order to reduce impurities therein to levels sufficiently low that the resulting ammonium alkanoyl alkyl sulfonate, e.g. ammonium cocoyl isethionate, has an acceptably low color and clear point to be used in clear liquid products. Two methods of controlling the pH during the reaction of ammonium bisulfite and alkylene oxide, e.g. ethylene oxide, are described in detail hereafter. One method involves lowering the initial pH of the reaction mixture of ammonium bisulfite and alkylene oxide. Another method involves adding an acidic neutralizing agent to the reaction mixture in response to increasing pH therein so as to maintain the pH within a prescribed range substantially throughout the reaction.

In a further related embodiment, the present invention relates to reacting ammonium hydroxyalkyl sulfonate and fatty acid in a metal-containing reaction vessel, e.g. a stainless steel autoclave, to produce ammonium alkanoyl alkyl sulfonate, and provides a method for preventing corrosion of the metal-containing reaction vessel, which can also cause contamination and discoloration of the reaction product. In this embodiment, the present invention comprises adding hypophosphorous acid (HPPA) or salts of HPPA to the reaction mixture of ammonium hydroxyalkyl sulfonate and fatty acid, the presence of which greatly reduces corrosion of the metal-containing reaction vessel, as determined by analysis for metal in the ammonium alkanoyl alkyl sulfonate reaction product.

### DETAILED DESCRIPTION OF THE INVENTION

Surface-active materials of the general formula, RCO(O)R'SO₃M wherein M is the ammonium cation, e.g., ammonium alkanoyl alkyl sulfonates, may be prepared by a process involving the direct esterification of a fatty acid with an ammonium hydroxyalkyl sulfonate, particularly ammonium isethionate in aqueous solution. In the preceding general formula, R represents the aliphatic hydrocarbon residue of a fatty acid containing from 6 to 24 carbon atoms, i.e. R is a monovalent aliphatic hydrocarbon radical containing from 5 to 23 carbon atoms, and R' represents a bivalent branched or straight chain hydrocarbon radical containing from 2 to 4 carbon atoms. Preferably, R is an aliphatic hydrocarbon radical having from 7 to 17 carbon atoms (fatty acid containing from 8 to 18 carbon atoms) for reasons of solubility and detergency. More preferably, R is an aliphatic hydrocarbon radical containing from 9 to 17 carbon atoms, and R' is the bivalent ethylene radical, i.e. (-CH₂-CH₂-).

The aliphatic hydrocarbon radical R includes linear and branched aliphatic radicals, and further includes mixtures of aliphatic radicals within the described carbon chain length, as are found, for example, in fatty acids derived from natural fats or oils. Fatty acids used in the present process may be prepared synthetically, but are conveniently available as the mixed fatty acids derived from naturally occurring vegetable fats and oils, such as coconut oil, palm oil, babassu oil, castor oil, olive oil, peanut oil, rape seed oil, corn oil, sesame seed oil, cotton seed oil, soybean oil, sunflower seed oil, safflower seed oil and hemp oil (hydrogenated and unhydrogenated). Lauric, caprylic, caproic, myristic, palmitic, stearic, palmitoleic and oleic acids also can be used, alone or in admixture, or in substitution for a part of the fatty acid reactant. Fatty acids derived from coconut oil, which comprise a mixture of principally C₈ to C₁₈ fatty acids, represent the preferred fatty acid reactant.

The ammonium salt of the hydroxyalkyl sulfonic acid used as a reactant in the process described herein may be represented by the general formula HOR'SO₃M, wherein R' and M are the same as defined hereinabove. More particularly, the ammonium hydroxyalkyl sulfonate reactant may be represented by the general formula 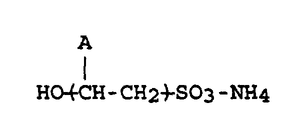 wherein A is selected from the group consisting of hydrogen, methyl and ethyl, preferably hydrogen, i.e. ammonium isethionate.

Copending U.S. patent application Serial No. 07/721,741, filed June 26, 1991, discloses that ammonium hydroxyalkyl sulfonate used in the above-described esterification process may be prepared by the reaction of ammonium bisulfite with an alkylene oxide containing from 2 to 4 carbon atoms, i.e. ethylene oxide, propylene oxide or butylene oxide, as described, for example, in U.S. Patent 2,820,818, and reports that commercially available ammonium bisulfite has been found to contain significant amounts of ammonium sulfate as an impurity, e.g. 2 percent ammonium sulfate in a 60 percent ammonium bisulfite solution. Ammonium sulfate present in the ammonium bisulfite reactant is also then present as an impurity in the ammonium hydroxyalkyl sulfonate prepared from such ammonium bisulfite. For example, ammonium isethionate prepared from commercially available ammonium bisulfite has been found to contain as much as 6.5 weight percent ammonium sulfate.

Copending U.S. patent application Serial No. 07/721,741 further discloses that ammonium hydroxyalkyl sulfonate, e.g. ammonium isethionate, preferably containing less than two weight percent ammonium sulfate, may be obtained by reacting pure sulfur dioxide with ammonia followed by reacting the resulting ammonium bisulfite with a C₂ to C₄ alkylene oxide, e.g. ethylene oxide. It also discloses that ammonium hydroxyalkyl sulfonate, e.g. ammonium isethionate, containing less than 2, preferably less than 1, weight percent of ammonium sulfate may be prepared by purifying commercially available ammonium bisulfite, or by purifying the ammonium hydroxyalkyl sulfonate prepared therefrom, for example by crystallizing ammonium isethionate. Such crystallized ammonium isethionate typically can be obtained with less than one weight percent ammonium sulfate.

When the condensation reaction of ammonium hydroxyalkyl sulfonate and fatty acid is carried out in a metal-containing reaction vessel, e.g. a stainless steel autoclave, corrosion of the vessel's metal occurs. To avoid such corrosion, the reaction can be conducted in a glass reaction vessel which limits the commercial practicability of the process. Metal corrosion of the reaction vessel also results in contamination of the ammonium alkanoyl alkyl sulfonate product with metallic corrosion products, e.g. iron, coming from the metal surface of the reactor. Iron contamination produces discoloration of the ammonium alkanoyl alkyl sulfonate product, making it less desirable for use in clear liquid compositions. Moreover, the iron contaminant also may interact with other ingredients in cosmetic compositions, e.g. perfumes.

The present invention provides a method for producing ammonium hydroxyalkyl sulfonate products, e.g. ammonium isethionate, from commercially available ammonium bisulfite, which products have sufficiently low levels of impurities for use, without crystallization, to produce ammonium alkanoyl alkyl sulfonates, e.g. ammonium cocoyl isethionate, which are sufficiently clear, especially at ambient temperatures, to be usable in clear liquid products. Moreover, the ammonium alkanoyl alkyl sulfonates can be produced in a commercially practicable reaction time. The method of the present invention for producing relatively pure ammonium hydroxyalkyl sulfonates, e.g. ammonium isethionate, involves controlling the pH of the reaction mixture of ammonium bisulfite and alkylene oxide, e.g. ethylene oxide, within the specified range of 4.3 to 7.0 substantially throughout the reaction. The pH of such reaction mixture increases naturally as the reaction proceeds. In accordance with the present invention, there are disclosed two embodiments for controlling the pH.

In one embodiment of the present invention, the initial pH of the reaction mixture of ammonium bisulfite and alkylene oxide, e.g. ethylene oxide, is brought into the range of 4.5 to 5.2, preferably 4.6 to 5.0, most preferably about 4.7 by the addition of a suitable acidic reagent. Lowering the initial pH to within the aforedescribed range reduces the proportion of ammonium ion available to react with alkylene oxide to produce alkanolamines. The pH of the reaction mixture will still increase as the reaction proceeds, but it will remain closer to neutral substantially throughout the reaction. Preferably, the pH will be maintained below about 7 until the reaction is at least 90 percent, preferably closer to 95 percent, complete as measured by the amount of residual sulfite.

In another embodiment of the present invention, the pH of the reaction mixture of ammonium bisulfite and alkylene oxide, e.g. ethylene oxide, which is typically in the range of 5.5 to 6.0, is maintained in the range of 5.5 to 7.0 substantially throughout the reaction, whereas the pH would otherwise increase above 7.0, by the addition, as required, of an acidic neutralizing agent in amounts sufficient to maintain the pH in the desired range substantially throughout the reaction cycle. The neutralizing agent may be an acid such as sulfurous acid, but is preferably sulfur dioxide, SO₂, which may be expeditiously added by bubbling SO₂ gas into the reaction mixture.

For example, an ammonium bisulfite solution is reacted with an equimolar amount of ethylene oxide; samples of the reaction mixture are taken periodically, analyzed for sulfite content and pH. When the pH of the reaction mixture rises to a level near or above 7, SO₂ is added, continuously or intermittently, to keep the pH between 6 and 7. Additional ethylene oxide is added to the reaction mixture in amounts that are stoichiometrically equivalent to the added SO₂. The reaction mixture is analyzed again for pH and sulfite content. Additional ethylene oxide is added, equivalent to the residual amount of sulfite; and this sequence is repeated until the residual sulfite level is acceptable, generally less than 0.35 weight percent, and preferably less than 0.2 weight percent. The reaction mixture is then vacuum stripped to remove any residual ethylene oxide, and the resulting ammonium isethionate can be reacted with coco fatty acid to produce ammonium cocoyl isethionate.

When ammonium bisulfite is reacted with alkylene oxide, e.g. ethylene oxide, to produce the corresponding ammonium hydroxyalkyl sulfonate product, e.g. ammonium isethionate, the corresponding alkanolamines and alkylene glycol, such as ethanolamines and ethylene glycol, are formed by the reaction of the alkylene oxide with ammonia or water, respectively, and are present as impurities in the product. For example, amounts from 1 to 4 weight percent of ethylene glycol and up to 12 weight percent ethanolamines have been found in ammonium isethionate prepared by reacting ethylene oxide with typical commercially available ammonium bisulfite.

In either of the above described embodiments, it is preferred to maintain the solids concentration of the reaction mixture of ammonium bisulfite and alkylene oxide, e.g. ethylene oxide, at less than 70 weight percent to maintain low levels of alkylene glycol in the ammonium hydroxyalkyl sulfonate product. Alkylene glycol, e.g. ethylene glycol, which is typically present as an impurity in such ammonium hydroxyalkyl sulfonate in amounts of from two to four weight percent, may be removed by vacuum stripping of the product. Vacuum stripping of the ammonium hydroxyalkyl sulfonate, e.g. ammonium isethionate, to remove alkylene glycol impurity will readily reduce the alkylene glycol level to less than 1 weight percent, e.g. less than 0.5 weight percent. For example, temperatures of about 140°C and pressures of 533 to 667 Pa [four to five millimeters (mm) of mercury (Hg)] are adequate to reduce the ethylene glycol level of ammonium isethionate to less than 0.5 weight percent. Low levels of alkylene glycol are maintained to minimize the reaction of alkylene glycol with fatty acid to form alkylene glycol esters which are relatively insoluble in water, and which therefore cloud solutions of the ammonium alkanoyl alkyl sulfonate product.

The presence of significant amounts of ammonium sulfate, alkanolamine and/or alkylene glycol impurities in the ammonium hydroxyalkyl sulfonate have been found to delay the onset of the reaction of fatty acid with the ammonium hydroxyalkyl sulfonate, thereby resulting in increased reaction times. Products of lower purity and anionic activity, e.g. less than 85 percent anionic activity, are also produced when such impurities are present. Such products have poor color and yield turbid aqueous solutions. For example, it has been observed that the reaction of coco fatty acid with an ammonium isethionate aqueous solution obtained by ethoxylation of commercially available ammonium bisulfite required 14 to 16 hours at 180°C to complete. The ammonium cocoyl isethionate product thus obtained was very dark, had a slight sulfide odor and gave dark turbid aqueous solutions. Further, the anionic activity of the product thus obtained was only in the range of 80 to 82 percent. The addition of conventional catalysts such as dodecylbenzenesulfonic acid, zinc oxide and quaternary ammonium compounds to the aforedescribed reaction was found to have no significant effect on reducing reaction times when significant amounts of ammonium sulfate and alkylene glycol impurities are present in the ammonium hydroxyalkyl sulfonate reactant.

In contrast, the reaction between coco fatty acid and ammonium isethionate produced in accordance with the present invention, which is thus devoid of significant amounts of impurities, i.e. having less than about one weight percent of ethylene glycol, two weight percent ethanolamine and two weight percent ammonium sulfate, has been found to proceed readily at 180°C. The reaction begins in one to two hours. Subsequently, the reaction mixture becomes homogeneous and water begins to distill. The reaction is completed in a total of about six to ten hours to produce good yields, e.g. 90 to 100 percent, of ammonium cocoyl isethionate having high purity, e.g. 90 to 97 percent. Aqueous solutions prepared from such a product are substantially clear, i.e. non-turbid.

In accordance with a preferred embodiment of the present invention, ammonium alkanoyl alkyl sulfonate, e.g. ammonium cocoyl isethionate, having an anionic activity of greater than 85 percent, e.g. 86 to 97 percent, is prepared by heating ammonium hydroxyalkyl sulfonate, e.g. ammonium isethionate, having less than 2, preferably less than 1, weight percent of ammonium sulfate, less than 2 weight percent alkanolamines, e.g. ethanolamines, and less than 1 weight percent alkylene glycol, e.g. ethylene glycol, with a fatty acid containing from 8 to 18 carbon atoms, e.g. coco fatty acid, at temperatures of from 150°C to 200°C.

In carrying out the preparation of ammonium alkanoyl alkyl sulfonates from an aqueous solution of ammonium hydroxyalkyl sulfonate, e.g. ammonium isethionate, it is common to employ at least a stoichiometric amount of the fatty acid reactant. Commonly, a molar excess of the monocarboxylic fatty acid is used, e.g. from 3 to 10 percent molar excess. However, an excess of the ammonium hydroxyalkyl sulfonate reactant may also be used. Thus, the mole ratio of fatty acid to ammonium hydroxyalkyl sulfonate will typically range from 0.95:1 to 1.1:1. Care should be observed to avoid a large excess of fatty acid since the presence of fatty acid and alkylene glycol esters, e.g. ethylene glycol esters, in the product can result in turbid aqueous solutions of the ammonium alkanoyl alkyl sulfonate, e.g. ammonium cocoyl isethionate, which are of limited use in clear cosmetic, e.g. shampoo, formulations. The amount of fatty acid impurity present in the final ammonium alkanoyl alkyl sulfonate product is commonly less than five weight percent, e.g. in the three to four weight percent range. Preferably, the amount of fatty acid impurity is less than three weight percent. The amount of alkylene glycol ester is preferably less than 2 weight percent, and more preferably less than 1 weight percent, to produce clear liquid products.

Reaction temperatures for the condensation reaction of ammonium hydroxyalkyl sulfonate and fatty acid are typically in the range of from 150 to 200°C. For the condensation of ammonium isethionate and coco fatty acid, condensation temperatures typically range from 150 to 190°C, e.g. about 180°C. At temperatures greater than 200°C, charring of the condensation product is likely to occur and, therefore, such high temperatures are to be avoided. Hence, temperatures below that at which charring of the ammonium alkanoyl alkyl sulfonate product occurs are used.

The condensation reaction is typically initiated under an inert gaseous atmosphere, e.g. a nitrogen purge, until by-product water, which results from the condensation reaction, has been removed, and the reaction mixture becomes homogeneous. Thereupon, a partial vacuum, i.e., 66,650 to 93,310 Pa [500 to 700 millimeters (mm) of mercury (Hg)], is applied to the reaction vessel to reduce the pressure to subatmospheric levels, and the reaction continued at the aforedescribed reaction temperatures until the reaction is essentially completed, whereupon a high vacuum, i.e., 667 to 1,333 Pa [5 to 10 mm of Hg], is applied to remove unreacted fatty acid. Thereafter, the vacuum is released, and the product removed from the reaction vessel.

The aforedescribed reaction proceeds readily in the absence of catalyst when significant levels (as described above) of alkanolamine, ammonium sulfate and alkylene glycol impurities are absent. Such reaction typically initiates within about one to two hours after attaining reaction temperature and is completed within six to ten hours. Optionally, catalytic amounts of conventional catalyst materials may be used to enhance the condensation reaction. Such catalysts include, without limitation, dodecylbenzenesulfonic acid (DDBSA), p-toluenesulfonic acid (PTSA), zinc oxide and quaternary ammonium compounds, such as stearyltrimethyl ammonium chloride. Mixtures of such catalysts may also be used. Catalytic amounts of such materials typically range from 0.1 to 3 weight percent, based on the weight of the reactants. The reaction mixture may further comprise a small quantity of previously formed ammonium alkanoyl alkyl sulfonate, e.g. ammonium cocoyl isethionate, referred to herein as a "heel", which may comprise up to 20 percent, preferably 5 to 12 percent, by weight of the total solids, i.e. the sum of ammonium hydroxyalkyl sulfonate, fatty acid and heel.

The ammonium alkanoyl alkyl sulfonate, e.g. ammonium cocoyl isethionate, product prepared in accordance with the process of the present invention may be obtained in yields of at least about 90 percent, and at least about 85 percent purity. The purity of the ammonium alkanoyl alkyl sulfonate product preferably ranges from 88 to 97 percent, e.g. 90 to 92 percent. The major impurity in the final product is typically unreacted fatty acid, which is preferably less than five weight percent, and which may be in the three to four weight percent range, but is most preferably less than three weight percent. The anionic activity of the product is at least 85 percent, and preferably is from 88 to 97 percent, based on solid ammonium alkanoyl alkyl sulfonate. Ammonium cocoyl isethionate having an anionic activity of less than 85 percent, based on solid ACI, forms turbid aqueous solutions which are not useful for the preparation of clear commercial formulations used in cosmetic products, such as clear shampoos. The anionic activity of the ammonium alkanoyl alkyl sulfonate products of the present process may be measured by ASTM Test Method D1681-83.

Ammonium alkanoyl alkyl sulfonates, such as ammonium cocoyl isethionate (ACI), are solids at room temperature. ACI is a stiff paste even at higher temperatures. Such products are, therefore, inherently difficult to remove from the reaction vessel in which they are prepared. While it would be convenient to remove such materials from the reaction vessel as a solution in water, materials such as ACI are unstable in aqueous solution under acidic conditions. Aqueous solutions of ammonium cocoyl isethionate generally have a pH of 3.0 to 3.5. Due to its acid pH, ACI hydrolyzes easily when dissolved in water in the reaction vessel at elevated temperatures, thereby resulting in the loss of product.

It is disclosed in copending U.S. patent application Serial No. 07/721,741 that solid ammonium alkanoyl alkyl sulfonate product may be removed readily from the reaction vessel by dissolution in water without significant hydrolysis if the natural acidity of the product is adjusted to within a pH range of from 6 to 8, preferably 6.5 to 7.5, e.g. 6.7 to 7.2. In adjusting the acidity of the product, it is preferred that the product first be cooled from reaction temperatures to about 120°C or less, e.g. from 50 to 120°C. The acidity of the ammonium alkanoyl alkyl sulfonate product may be adjusted with any suitable alkaline reagent, such as organic amines, e.g., triethanolamine, and ammonium hydroxide, preferably a substantially water-free, more preferably, anhydrous, alkaline reagent. Preferably, the alkaline reagent is relatively high boiling, i.e., relatively non-volatile at the temperature at which the product acidity is adjusted. Further, it is preferred that the alkaline reagent is one that is also acceptable for use in cosmetic applications. After adjustment of the acidity, sufficient water may be mixed with the "neutral" ammonium alkanoyl alkyl sulfonate product to dissolve it, form an aqueous solution thereof, and remove the product from the reactor.

The addition of an alkaline reagent and preferably subsequent addition of sufficient water to dissolve the product are performed with vigorous agitation to avoid localized conditions of high alkalinity (or high acidity) in the reaction product and to assist in the formation of an aqueous solution that may be readily removed from the reactor. Because the cooled product is in the form of a solid or stiff paste, several hours of stirring are required to dissolve all of the solid product. The resultant aqueous solution usually will have a product concentration of about 40 weight percent or less. Concentrations of from 25 to 35 weight percent are preferred to avoid gelling of the product. Because the ammonium hydroxyalkyl sulfonate, e.g. ammonium isethionate, prepared in accordance with the controlled pH method of the present invention contains low levels of alkanolamines, alkylene glycol and ammonium sulfate, the ammonium alkanoyl alkyl sulfonate produced therefrom forms clear, i.e. non-turbid, solutions at room temperature at these concentrations.

When using a metallic reaction vessel to conduct the ammonium hydroxyalkyl sulfonate-fatty acid condensation reaction, corrosion of the reaction vessel and the subsequent discoloration of the condensation product are inhibited by incorporating into the reaction mixture a corrosion-inhibiting amount of hypophosphorous acid (HPPA) or water-soluble salts thereof. The amount of HPPA used depends on a number of factors such as reaction vessel material, pH and concentration of reactants in the reaction mixture, impurities in reactants, temperature, duration of reaction, identity and amount of catalyst, and the like. Typically, from 0.01 to 1.0 weight percent, preferably from 0.02 to 0.5 weight percent, of hypophosphorous acid, based on the weight of the reactants, is sufficiently corrosion-inhibiting. The presence of HPPA in the reaction mixture of ammonium hydroxyalkyl sulfonate and fatty acid to form ammonium alkanoyl alkyl sulfonate does not appear to affect the esterification reaction or the levels of impurities other than metal impurities from corrosion of the metallic reaction vessel, e.g., a stainless steel autoclave.

While hypophosphorous acid is used in the following relevant examples, various salts of hypophosphorous acid may also be used. For example, alkali metal salts, such as sodium or potassium hypophosphite, may be used. Ammonium hypophosphite may also be suitable. Amine salts of hypophosphorous acid may also be employed, as well as alkanolamine salts of hypophosphorous acid, so long as there are no adverse reactions. Hypophosphorous acid is preferred, and is generally added as an aqueous solution, typically at about 50 weight percent concentration. The concentrations of HPPA in the examples are calculated as weight percent anhydrous HPPA, based on the solid weight of the reactants, i.e. ammonium hydroxyalkyl sulfonate, fatty acid, and, if present, ammonium alkanoyl alkyl sulfonate heel.

In the examples, hypophosphorous acid is added in a single step. However, in the practice of the present invention on a larger scale, it may be preferred to add the corrosion-inhibiting compound, preferably HPPA, in increments at several stages of the process to ensure that active HPPA is available in the latter phase of the reaction. For example, the selected amount of HPPA may be divided into several portions, not necessarily equal, and added to the reaction vessel at different stages of the process. One stage at which HPPA may be added is before the water is evaporated from the ammonium hydroxyalkyl sulfonate, e.g. ammonium isethionate, solution. Another stage for addition of HPPA is after the water has been evaporated from the ammonium hydroxyalkyl sulfonate, e.g. ammonium isethionate, solution but before the reaction mixture is further heated to effect the esterification reaction between the ammonium hydroxyalkyl sulfonate, e.g. ammonium isethionate, and the fatty acid, e.g. coco fatty acid. A portion of the HPPA may also be added after the esterification reaction of ammonium hydroxyalkyl sulfonate and fatty acid to produce ammonium alkanoyl alkyl sulfonate is substantially complete, but before the excess fatty acid, e.g. coco fatty acid, is stripped. More or fewer portions of HPPA may be utilized, and may be added at other stages so long as corrosion of the reaction vessel is effectively inhibited.

### Example 1

A one liter stainless steel autoclave was charged with 300 grams of ammonium bisulfite solution (57.1% assay NH₄HSO₃) having a pH of 4.94 and 211 grams of deionized water. The autoclave was pressure purged with nitrogen to remove air. The autoclave was heated to from 35 to 40°C, and 76 grams of ethylene oxide was added in portions as needed to keep the temperature at about 35°C with cooling water on, and the pressure less than 35 pounds per square inch gage (psig) (241 kPa). When the addition of ethylene oxide was completed, the temperature was maintained at approximately 35°C for several hours. Samples of the ammonium isethionate reaction mixture were taken during this period and analyzed for pH and sulfite, SO₃⁼, content with the following results.

| Hours | pH | Weight % SO₃⁼ |
|---|---|---|
| 1.5 | 7.1 | 2.3 |
| 2.5 | 8.4 | 0.63 |
| 3.5 | 8.4 | 0.34 |

The first sample above indicated that the reaction was about 91.5 percent complete before the pH increased to above 7, as calculated from the weight percent residual sulfite. Following analysis of the last sample, vacuum was applied to the reaction mixture for 45 minutes to strip out excess ethylene oxide. The stripped reaction mixture was analyzed and found to contain, by weight, 0.13 percent sulfite, SO₃⁼, 0.29 weight percent ethylene glycol, and 47.7 percent solids. Analysis by ¹³C NMR showed the presence of 0.9 mole percent ethanolamine.

Into a stirred reaction flask were placed 304 grams of the above aqueous ammonium isethionate (-1 mole), 233 grams of coco fatty acid (1.1 mole), and 88 grams of previously prepared aqueous ammonium cocoyl isethionate (31.0 weight percent solids in aqueous solution). The fatty acid used in all of the examples herein is C-108 fatty acid from the Procter & Gamble Co. The reaction mixture was heated to 110°C, and the water was removed by vacuum stripping for 45 minutes under approximately 13,330 Pa (100 mm Hg) pressure.

The reaction flask was opened, 1 gram of p-toluenesulfonic acid monohydrate catalyst was added, the flask was resealed and heated to 150 to 160°C with a nitrogen purge. After 3.5 hours, the reaction mixture coupled to become one phase, and the temperature was maintained for one additional hour. Then excess fatty acid was removed by vacuum stripping at approximately 1,333 Pa (10 mm Hg) pressure for 4 hours at the same temperature.

The vacuum was released with nitrogen, and the reaction mixture cooled to 110°C. An aqueous solution of 1.5 grams concentrated ammonium hydroxide in 800 grams of water was added, and the mixture was stirred at 50 to 60°C until completely dissolved. The final solution had a pH of 6.1, 30.9 percent solids, with an anionic activity of 27.7 percent (89.6 percent anionic activity on a 100 percent solids basis). When chilled to become cloudy and subsequently warmed, the solution became clear at 15°C (clear point).

### Example 2

Commercially available ammonium bisulfite solution (60.9 percent assay NH₄HSO₃) was obtained from the manufacturer, P.B.& S. Chemical Company, Inc. Sulfur dioxide gas was bubbled into the solution at 10°C until the pH dropped to 4.8.

A stainless steel autoclave was charged with 800 grams of the above solution and 232 grams of deionized water. The vapor space of the reactor was purged with nitrogen to remove air, and the autoclave was heated to from 35 to 40°C. 220 grams of ethylene oxide (EO) were then added, keeping the temperature at from about 35 to 40°C. Following completion of the addition of EO, the reaction was continued for 1 hour, and the ammonium isethionate reaction mixture was sampled and found to contain 3.9 weight percent residual sulfite anion, and have a pH of 5.7. An additional 5 grams of ethylene oxide were added, and the reaction was continued for 1 hour. Analysis of a sample of the reaction mixture revealed a pH of 6.0 and a residual sulfite anion content of 2.3 weight percent, indicating that the reaction had proceeded 94.3 percent toward completion. An additional 10 grams of ethylene oxide was added, followed by an additional 1 hour reaction period. Analysis of the resulting reaction mixture showed that the sulfite anion content had dropped to 0.47 weight percent, and the pH had increased to 7.7. No additional ethylene oxide was added. The reaction was continued for 1 hour, and a further analysis showed that the sulfite content had dropped to 0.3 weight percent. The reaction mixture had a pH of 7.76. After 1 final hour of reaction time, the reaction mixture was vacuum stripped to remove any residual ethylene oxide, and a final analysis of the reaction mixture indicated a residual sulfite anion content of 0.24 weight percent, a pH of 8.4, a solids content of 60.5 weight percent and an ethylene glycol content of 0.68 weight percent. By ¹³C NMR analysis, 0.4 mole percent ethanolamine was also found to be present.

Into a stirred reaction flask were placed 247 grams of the above produced aqueous ammonium isethionate, 223 grams of coco fatty acid, and 80 grams of previously prepared aqueous ammonium cocoyl isethionate solution (30.9 percent solids). The reaction mixture was heated to 110°C and most of the water was stripped off. The reaction flask was opened, 1 gram of p-toluenesulfonic acid monohydrate catalyst was added, the flask was resealed and heated to 150 to 160°C with a nitrogen purge. After 7.5 hours at the above temperature, the excess fatty acid was stripped off under vacuum for 4 additional hours at 1,333 Pa (10 mm of Hg) or less.

The vacuum was released with nitrogen and the reaction mixture was cooled to 110°C. An aqueous solution of 1.2 grams of concentrated ammonium hydroxide in 800 grams of water was added and the mixture was stirred until completely dissolved. The final solution had an anionic activity of 27.7 percent at a 31.0 percent solids concentration (89.4 percent anionic activity on a 100 percent solids basis). When chilled to become cloudy and subsequently warmed, the solution became clear at 19°C (clear point).

### Comparative Example A

To illustrate the effect of uncontrolled high pH in the preparation of ammonium isethionate on the ammonium cocoyl isethionate prepared therefrom, commercially available ammonium bisulfite solution (57.9 percent assay NH₄HSO₃) was obtained from the manufacturer, P.B.& S. Chemical Company, Inc., and the pH of the solution, 5.5 as received, was not controlled in accordance with the method of the invention. A one gallon stainless steel autoclave was charged with 1680 grams of the ammonium bisulfite solution and 1228 grams of deionized water. The autoclave was purged twice with nitrogen in order to remove air. The ammonium bisulfite solution was heated to 30°C, and 430 grams of ethylene oxide (EO) added in amounts as required to maintain the temperature at 30 to 35°C and the pressure below 40 psig (276 kPa). After all the EO had been added, the reaction was continued for one hour. The reaction mixture was analyzed and found to contain a high level of residual sulfite anion. Three additions of ethylene oxide (30, 30 and 60 grams) were made to the reaction mixture. Finally, the reaction mixture was vacuum stripped to remove any unreacted ethylene oxide. The ammonium isethionate reaction product contained 0.91 weight percent sulfite anion and 0.36 weight percent ethylene glycol. Analysis by ¹³C NMR indicated the presence of 14.7 mole percent ethanolamine, 4.8 mole percent diethanolamine and 0.9 mole percent triethanolamine.

The above ammonium isethionate solution contained 47.8 percent solids. A stirred reaction flask was charged with 192 grams of this ammonium isethionate solution (0.6 moles). The solution was heated to 36°C under nitrogen, then vacuum stripped at 8 millimeters mercury for 45 minutes. To this solution were added 138 grams (10 percent molar excess) of coco fatty acid and 0.12 gram of 50 percent aqueous hypophosphorous acid (HPPA). The reaction mixture was gradually heated to 110°C and held at that temperature until the distillation of water slowed. The reaction mixture was then vacuum stripped at about 5 millimeters of mercury for 2 hours. An additional 0.2 gram of HPPA was added, and the temperature was slowly increased to 175°C. The reaction mixture was held at 175°C for 6.4 hours then cooled to about 150 to 160°C. An additional 0.19 gram of HPPA was added, and the reaction mixture was vacuum stripped at about 800 Pa (6 millimeters of mercury) for 4 hours. After cooling to about 30°C under nitrogen, the very hard solid product was broken up with a spatula and dissolved in 410 grams of water containing 1.2 grams of triethanolamine (TEA), while maintaining the temperature of the solution at about 50 to 60°C and the pH at 7±0.5 by addition of TEA as needed. An additional 3.34 grams of TEA was needed.

The final solution of ammonium cocoyl isethionate had a pH of 7.2, contained 31.4 percent solids, and had an anionic activity of 19.0 percent (60.5 percent anionic activity on a 100 percent solids basis). The solution was thick and cloudy at ambient temperature, and did not become clear upon warming until it reached 38°C (clear point), which is an unacceptably high clear point for use in clear liquid compositions.

### Comparative Example B

This comparative example illustrates the effect of high sulfate content in ammonium bisulfite and the ammonium isethionate produced therefrom on the ammonium cocoyl isethionate produced from such ammonium isethionate. A 70 percent aqueous solution of typical commercially available ammonium bisulfite (566.0 grams) was diluted with 225 milliliters (ml) of water and charged to a one liter autoclave. The autoclave was sealed under a positive nitrogen pressure of 10 psig (69 kPa), and the solution heated to 60°C. Ethylene oxide (EO) was added to the solution in the autoclave at a rate of about 2 grams per minute. The addition of ethylene oxide was stopped after 180 grams had been added to the autoclave. The pressure within the autoclave during most of the EO addition was 30 to 35 psig (207 to 241 kPa). The resultant reactant mixture was stirred at 60°C for 1.5 hours and then cooled to 40°C. The pressure within the autoclave was released and the contents weighed. The weight gain was 175 grams and the pH of the solution was 8.4. The solution had a solids content of 60 percent and a sulfate content of 4.7 percent, which corresponded to 6.5 weight percent ammonium sulfate.

An aqueous solution of ammonium isethionate (206 grams) prepared as above containing about 6.5 weight percent ammonium sulfate was charged to a reaction vessel equipped with mechanical stirrer, temperature controller and nitrogen inlet. The solution was heated under nitrogen to 140°C to remove water. When the distillation of water stopped, the reactor was evacuated gradually to a subatmospheric pressure of 1,333 Pa (10 mm of mercury) to remove volatiles. The vacuum was released and 209 grams of coco fatty acid and 5 drops of dodecylbenzenesulfonic acid (DDBSA) catalyst were added to the residue in the reaction vessel. The reaction mixture was heated to 180°C and stirred for 3.25 hours under nitrogen. No reaction occurred during this period. The heat to the reaction vessel was turned off and the reaction mixture allowed to stand at room temperature overnight (about 16 hours).

The reaction mixture was then reheated to 180°C and stirred under nitrogen for 2 hours. No reaction was observed. P-toluenesulfonic acid (PTSA) catalyst (0.2 grams) was then added to the reaction mixture, and the mixture stirred for 2 hours at 180°C. No reaction was observed to occur as evidenced by the absence of a water distillate. Stearyl trimethyl ammonium chloride (0.1 grams) was then added to the reaction mixture, and the mixture heated for 1 hour at 180°C. Significant reaction was still not observed. The reaction vessel was gradually evacuated to a pressure of 1,333 Pa (10 mm of mercury) and maintained at 180°C. The reaction mixture became homogeneous and thick after 4 hours under vacuum at 180°C. The vacuum on the reactor was then released, and the product removed. The total reaction time at 180°C was 14 hours. The anionic activity of the solid product was found to be 82.3 percent. The product was dark in color, and when a 30 percent aqueous solution was prepared from the product, a turbid solution was formed.

### Comparative Example C

This comparative example illustrates the effect of ethylene glycol in the ammonium isethionate on the ammonium cocoyl isethionate (AIS) produced therefrom. A 500 milliliter, 3-necked flask was charged with 120 grams of an aqueous ammonium isethionate solution (60 percent solids), which contained 0.8 weight percent of ammonium sulfate and about 3.7 weight percent of ethylene glycol, and 109.5 grams of C-108 coco fatty acid. The mixture was heated with stirring under a nitrogen sparge to 170°C over a period of 1.75 hours. Most of the water contained in the reaction mixture was distilled during this time. The reaction mixture was maintained at 170°C for 7 hours before water from the condensation reaction began to distill. The reaction mixture was maintained at 170°C for an additional 3 hours. During this period, 50 milliliters of water and 12 milliliters of fatty acid were collected as distillates. Heat to the reactor was turned off, and the mixture allowed to stand overnight at room temperature. Distilled fatty acid was returned to the reactor and the mixture reheated with stirring to 170°C and maintained at such temperature for 2 hours. The reaction mixture was then slowly evacuated to a pressure of 533 to 667 Pa (4 to 5 millimeters of mercury) and maintained at such pressure for 2 hours at 170°C. The mixture was then cooled and the reaction product removed. Anionic activity of the product was found to be 80.2 percent. A 30 percent aqueous solution of the product was prepared. The solution appeared turbid.

### Example 3

These examples demonstrate what levels of ethylene glycol and ethanolamine impurities (added to crystalline ammonium isethionate (AIS), which contains insignificant amounts of these impurities) produce ACI with an unacceptably high clear point in solution.

A reaction flask was charged with 71.6 grams (0.5 moles) of crystalline ammonium isethionate, 114.8 grams (0.55 moles, 10% excess over stoichiometric) of coco fatty acid. 0.5 grams of p-toluenesulfonic acid monohydrate, and the impurity to be tested.

The nitrogen flow was started, with stirring, and the reaction mixture was heated to 150 to 160°C and held at that temperature. After a period of time (ranging from 1 hour 10 minutes to 9 hours 25 minutes, depending on the impurity and the amount added), the two phases of the reaction mixture "coupled", i.e. became a homogeneous single phase.

The reaction mixture was maintained at 150 to 160°C for an additional 2 to 4 hours as the viscosity increased and the reaction mixture developed a "dough-like" consistency. Excess unreacted fatty acid was removed by vacuum stripping the reaction mixture for 2.5 to 4 hours at 4 to 8 mm of Hg pressure and 150 to 160°C. After cooling, the reaction mixture was broken into small pieces, samples analyzed for anionic activity, and dissolved in water.

The solidified reaction mixture was stirred with deionized water at 50 to 60°C. The pH was monitored with a pH electrode and the pH was kept at about pH 7 (± 0.5) by dropwise addition of triethanolamine as needed. Once the solids were completely dissolved, a final adjustment in concentration was made, if needed, by addition of water.

The clear point was measured by chilling the sample until it became cloudy, then allowing the sample to warm slowly. The clear point was measured as the temperature at which the sample became clear, or essentially clear (with just a very slight haze which did not disappear on further warming). The data in Table I show that as the level of glycol in the ammonium isethionate approaches 0.9 percent, and the level of alkanolamine 2 percent, the clear point of the ammonium cocoyl isethionate (ACI) produced therefrom approaches ambient temperature. Therefore, to provide an ammonium alkanoyl alkyl sulfonate used should be which is clear at ambient temperature, the ammonium hydroxyalkyl sulfonate used should be substantially pure, i.e. have levels of these impurities below these amounts.

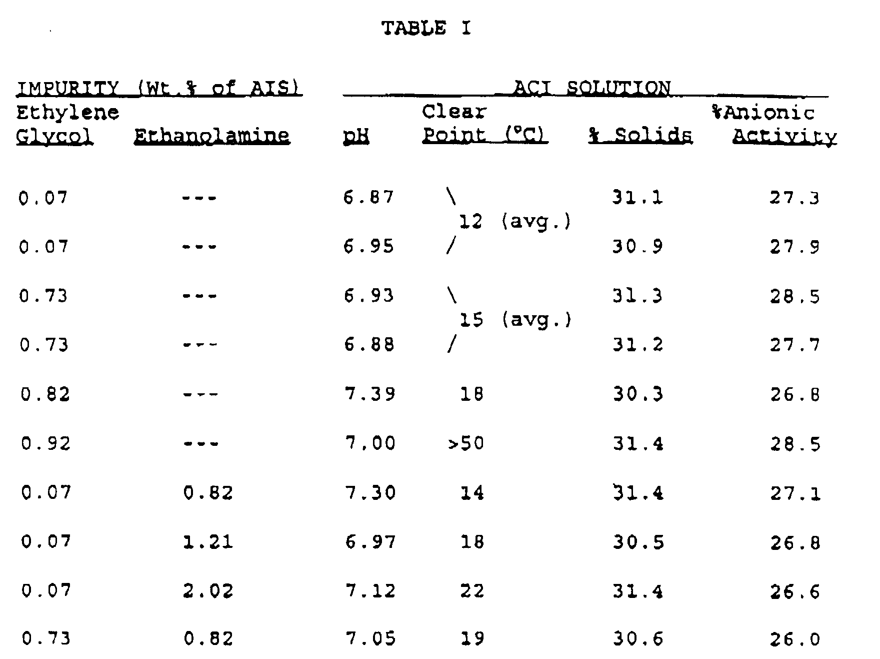

### Comparative Example D

A stainless steel Parr reactor was charged with 321 grams of ammonium isethionate (44 percent aqueous solution), 223 grams of coco fatty acid and a heel of 92 grams of ammonium cocoyl isethionate (23.9 percent aqueous solution). The reactants were heated to 105°C in a nitrogen atmosphere and vacuum stripped before adding 1 gram of para-toluene sulfonic acid catalyst (PTSA). The reaction mixture was heated to 150 to 160°C for 4 hours, cooled and allowed to stand in the reactor overnight. The next morning, the reaction mixture was reheated to 150 to 160°C and vacuum stripped at 1,333 Pa (10 mm Hg) to remove excess fatty acid. Samples were taken periodically and analyzed for iron content. The initial sample, taken before heating, had 1.4 ppm iron. The second sample, taken after heating at 105°C but before adding the catalyst, contained 16 ppm iron. The third sample, taken after 4 hours heating at 150 to 160°C, had 50 to 80 ppm iron. The final sample, taken after the reaction mixture had been in the reactor overnight and vacuum stripped of excess fatty acid, contained more than 400 ppm iron. If diluted to a 30 percent solids solution, the concentration of iron would be more than 120 ppm.

### EXAMPLE 4

A reaction mixture was prepared and reacted as in the previous Comparative Example D, except that 0.16 percent hypophosphorous acid (based on anhydrous weights of hypophosphorous acid and reactants, including ACI heel) was added. The hypophosphorous acid (HPPA) was added as a 50 percent aqueous solution. The HPPA was added before the water from the ammonium isethionate solution was evaporated. The first sample, taken after stirring the reaction mixture for 35 minutes, had 1.9 ppm iron. After vacuum stripping off water, the reaction mixture contained 3.0 ppm iron before addition of the catalyst. After 4 hours heating with the added catalyst, the reaction mixture contained 10 ppm iron. The final sample, after 4 hours of vacuum stripping, contained 34 ppm iron. As diluted to 30 percent solids, the final reaction mixture contains 10 ppm iron.

### EXAMPLE 5

A second reaction mixture prepared as in Example 4, except for adding 0.13 weight percent HPPA, contained 1 ppm iron before adding the catalyst, 13 ppm iron before coupling, 27 ppm iron after coupling, and 20 ppm iron in the final undiluted sample, which calculated to 6 ppm iron for a 30 percent solids composition.

The preceding examples are offered to illustrate the present invention. Various modifications of the invention are included within the scope as defined by the following claims.

## Claims

1. A method for producing ammonium alkanoyloxy alkyl sulfonate of the general formula I
RCO(O)R'SO₃ M (I)
wherein R is a monovalent hydrocarbon residue of a fatty acid containing from 6 to 24 carbon atoms, R' is a bivalent hydrocarbon radical containing from 2 to 4 carbon atoms, and M is the ammonium cation, comprising:
(a) reacting an aqueous solution of ammonium bisulfite with an alkylene oxide containing from 2 to 4 carbon atoms while maintaining the solids concentration of the reaction mixture at less than 70 weight percent and the pH of the reaction mixture in the range of 4.3 to 7 until the reaction is at least 90 percent complete, as measured by the amount of residual sulfite in the reaction mixture, thereby to produce a product consisting essentially of ammonium hydroxyalkyl sulfonate of the general formula II
HOR'SO₃ M (II)
wherein R' and M are as defined above, said product containing alkylene glycol by-product;
(b) stripping ammonium hydroxyalkyl sulfonate prepared in (a) to remove alkylene glycol; and
(c) reacting ammonium hydroxyalkyl sulfonate having not more than 1 weight percent alkylene glycol, not more than 2 weight percent alkanolamine, and not more than about 2 weight percent ammonium sulfate, all based on the weight of the ammonium hydroxyalkyl sulfonate, with a fatty acid containing from 6 to 24 carbon atoms at a temperature in the range of from 150 to 200°C, thereby to produce an ammonium alkanoyloxy alkyl sulfonate of the above general formula, a 25 to 35 weight percent aqueous solution of which is non-turbid at room temperature.

2. The method of claim 1 wherein the bivalent hydrocarbon radical, R', is the ethylene bivalent radical, and the alkylene oxide is ethylene oxide.

3. The method of claim 1 or 2 wherein the fatty acid contains from 8 to 18 carbon atoms.

4. The method of any of claims 1 to 3 wherein the initial pH of the ammonium bisulfite solution prior to reaction with alkylene oxide is in the range of 4.5 to 5.2.

5. The method of claim 4 wherein the pH is in the range from 4.6 to 5.0.

6. The method of any of claims 1 to 3 wherein the pH of the reaction mixture in part (a) is maintained within the range of 5.5 to 7.0 substantially throughout the reaction by the addition of an acidic neutralizing agent.

7. The method of claim 6 wherein the acidic neutralizing agent is sulfurous acid or SO₂.

8. The method of any of claims 1 to 7 wherein the ammonium hydroxyalkyl sulfonate used in part (c) contains not more than 0.85 weight percent alkylene glycol.

9. The method of any of claims 1 to 8 wherein the ammonium alkanoyloxy alkyl sulfonate has an anionic activity of at least 85 percent.

10. The method of any of claims 1 to 9 wherein the pH of the reaction mixture is maintained below about 7 during the reaction of part (a) by (i) establishing the initial pH of the ammonium bisulfite reaction mixture in the range of 4.5 to 5.2, or (ii) adding an acidic neutralizing agent to the reaction mixture as required to maintain the pH within the range of 5.5 to 7.

11. The method of claim 10 wherein the fatty acid is coco fatty acid.

12. The method of any of claims 1 to 11 wherein the ammonium alkanoyloxy alkyl sulfonate has an anionic activity of greater than 85 percent, and wherein a 25 to 35 weight percent aqueous solution of said ammonium alkanoyloxy alkyl sulfonate is not turbid at room temperature.

13. The method of any of claims 1 to 12 wherein the ammonium hydroxyalkyl sulfonate used in part (c) contains less than 0.5 weight percent ethylene glycol.

14. The method of any of claims 1 to 13 wherein the ammonium hydroxyalkyl sulfonate used in part (c) contains less than 1 weight percent ammonium sulfate

15. A clear liquid detergent composition comprising an aqueous solution of the ammonium alkanoyloxy alkyl sulfonate of any of claims 1 to 14.

16. The clear liquid detergent composition of claim 15 wherein the ammonium alkanoyloxy alkyl sulfonate is ammonium cocoyl isethionate.

## Patentansprüche

1. Verfahren zur Herstellung von Ammoniumalkanoyloxyalkylsulfonat der allgemeinen Formel I:
RCO(O)R'SO₃M (I),
worin R ein einbindiger Kohlenwasserstoffrest einer Fettsäue mit 6 bis 24 Kohlenstoffatomen ist, R' ein zweibindiger Kohlenwasserstoffrest mit 2 bis 4 Kohlenstoffatomen ist und M das Ammoniumkation ist, umfassend:
(a) Umsetzen einer wäßrigen Lösung von Ammoniumbisulfit mit einem Alkylenoxid, das 2 bis 4 Kohlenstoffatome enthält, während die Feststoffkonzentration der Reaktionsmischung auf weniger als 70 Gew.-% und der pH der Reaktionsmischung im Bereich von 4,3 bis 7 gehalten wird, bis die Reaktion zu wenigstens 90 % vervollständigt ist, gemessen durch die Menge an Restsulfit in der Reaktionsmischung, wodurch ein Produkt gebildet wird, das im wesentlichen aus Ammoniumhydroxyalkylsulfonat der allgemeinen Formel II besteht:
HOR'SO₃M (II),
worin R' und M wie oben definiert sind, und dieses Produkt Alkylenglykol als Nebenprodukt enthält,
(b) Strippen des in (a) hergestellten Ammoniumhydroxyalkylsulfonats, um Alkylenglykol zu entfernen, und
(c) Umsetzen des Ammoniumhydroxyalkylsulfonats, das nicht mehr als 1 Gew.-% Alkylenglykol, nicht mehr als 2 Gew.-% Alkanolamin und nicht mehr als ungefähr 2 Gew.-% Ammoniumsulfat aufweist, wobei diese Werte auf das Gewicht des Ammoniumhydroxyalkylsulfonats bezogen sind, mit einer Fettsäure, die 6 bis 24 Kohlenstoffatome enthält, bei einer Temperatur im Bereich von 150 bis 200° C, um dadurch ein Ammoniumalkanoyloxyalkylsulfonat der oben angegebenen allgemeinen Formel herzustellen, wobei eine 25- bis 35-gewichtsprozentige wäßrige Lösung davon bei Raumtemperatur nicht trübe ist.

2. Verfahren nach Anspruch 1, wobei der zweibindige Kohlenwasserstoffrest R' der zweibindige Rest Ethylen ist und das Alkylenoxid Ethylenoxid ist.

3. Verfahren nach Anspruch 1 oder 2, wobei die Fettsäure 8 bis 18 Kohlenstoffatome enthält.

4. Verfahren nach einem der Ansprüche 1 bis 3, wobei der anfängliche pH der Ammoniumbisulfitlösung vor der Reaktion mit dem Alkylenoxid im Bereich von 4,5 bis 5,2 liegt.

5. Verfahren nach Anspruch 4, wobei der pH im Bereich von 4,6 bis 5,0 liegt.

6. Verfahren nach einem der Ansprüche 1 bis 3, wobei der pH der Reaktionsmischung in Stufe (a) während im wesentlichen der gesamten Reaktion durch die Zugabe eines sauren Neutralisationsmittels innerhalb eines Bereichs von 5,5 bis 7,0 gehalten wird.

7. Verfahren nach Anspruch 6, wobei das saure Neutralisationsmittel schweflige Säure oder SO₂ ist.

8. Verfahren nach einem der Ansprüche 1 bis 7, wobei das Ammoniumhydroxyalkylsulfonat, das in Stufe (c) verwendet wird, nicht mehr als 0,85 Gew.-% Alkylenglykol enthält.

9. Verfahren nach einem der Ansprüche 1 bis 8, wobei das Ammoniumalkanoyloxyalkylsulfonat eine anionische Aktivität von wenigstens 85 % aufweist.

10. Verfahren nach einem der Ansprüche 1 bis 9, wobei der pH der Reaktionsmischung während der Reaktion der Stufe (a) unterhalb ungefähr 7 durch (i) Einstellen des anfänglichen pH's der Ammoniumbisulfitreaktionsmischung in einem Bereich von 4,5 bis 5,2 oder (ii) Zugabe eines sauren Neutralisationsmittels zu der Reaktionsmischung, wie es benötigt wird, um den pH innerhalb eines Bereichs von 5,5 bis 7 zu halten, gehalten wird.

11. Verfahren nach Anspruch 10, wobei die Fettsäure Kokosölfettsäure ist.

12. Verfahren nach einem der Ansprüche 1 bis 11, wobei das Ammoniumalkanoyloxyalkylsulfonat eine anionische Aktivität von mehr als 85 % aufweist und wobei eine 25- bis 35-gewichtsprozentige wäßrige Lösung dieses Ammoniumalkanoyloxyalkylsulfonats bei Raumtemperatur nicht trübe ist.

13. Verfahren nach einem der Ansprüche 1 bis 12, wobei das Ammoniumhydroxyalkylsulfat, das in Stufe (c) verwendet wird, weniger als 0,5 Gew.-% Ethylenglykol enthält.

14. Verfahren nach einem der Ansprüche 1 bis 13, wobei das Ammoniumhydroxyalkylsulfat, das in Stufe (c) verwendet wird, weniger als 1 Gew.-% Ammoniumsulfat enthält.

15. Klare flüssige oberflächenaktive Zusammensetzung, enthaltend eine wäßrige Lösung des Ammoniumalkanoyloxyalkylsulfonats nach einem der Ansprüche 1 bis 14.

16. Klare flüssige oberflächenaktive Zusammensetzung nach Anspruch 15, wobei das Ammoniumalkanoyloxyalkylsulfonat Ammoniumcocoylisethionat ist.

## Revendications

1. Procédé de production d'alcanoyloxy alkyl sulfonate d'ammonium de la formule générale I :
RCO(O)R'SO₃M (I)
dans laquelle R est un résidu hydrocarboné monovalent d'un acide gras contenant de 6 à 24 atomes de carbone, R' est un radical hydrocarboné bivalent contenant de 2 à 4 atomes de carbone et M est le cation ammonium, comprenant:
(a) la réaction d'une solution aqueuse de bisulfite d'ammonium avec un oxyde d'alkylène contenant de 2 à 4 atomes de carbone tout en maintenant la concentration en matières solides du mélange réactionnel à moins de 70% en poids et le pH du mélange réactionnel dans la gamme de 4,3 à 7 jusqu'à ce que la réaction soit complète au moins à 90%, telle que mesurée par la quantité de sulfite résiduel dans le mélange réactionnel, en produisant ainsi un produit composé essentiellement d'hydroxyalkyl sulfonate d'ammonium de la formule générale II :
HOR'SO₃M (II)
dans laquelle R' et M sont tels que définis précédemment, ledit produit contenant un sous-produit d'alkylène glycol;
(b) l'extraction de l'hydroxyalkyl sulfonate d'ammonium préparé dans (a) pour séparer l'alkylène glycol; et
(c) la réaction d'hydroxyalkyl sulfonate d'ammoniun ne contenant pas plus de 1% en poids d'alkylène glycol, pas plus de 2% en poids d'alcanolamine et pas plus d'environ 2% en poids de sulfate d'ammonium, le tout par rapport au poids de l'hydroxyalkyl sulfonate d'ammonium, avec un acide gras contenant de 6 à 24 atomes de carbone à une température dans l'intervalle de 150 à 200°C, en produisant ainsi un alcanoyloxy alkyl sulfonate d'ammonium de la formule générale ci-dessus, dont une solution aqueuse de 25 à 35% en poids est non trouble à la température ambiante.

2. Procédé suivant la revendication 1, dans lequel le radical hydrocarboné bivalent R' est le radical bivalent éthylène et l'oxyde d'alkylène est l'oxyde d'éthylène.

3. Procédé suivant l'une ou l'autre des revendications 1 et 2, dans lequel l'acide gras contient de 8 à 18 atomes de carbone.

4. Procédé suivant l'une quelconque des revendications 1 à 3, dans lequel le pH initial de la solution de bisulfite d'ammonium avant la réaction avec l'oxyde d'alkylène se situe dans la gamme de 4,5 à 5,2.

5. Procédé suivant la revendication 4, dans lequel le pH se situe dans la gamme de 4,6 à 5,0.

6. Procédé suivant l'une quelconque des revendications 1 à 3, dans lequel le pH du mélange réactionnel dans la partie (a) est maintenu dans la gamme de 5,5 à 7,0 pratiquement tout au long de la réaction par l'addition d'un agent neutralisant acide.

7. Procédé suivant la revendication 6, dans lequel l'agent neutralisant acide est de l'acide sulfureux ou SO₂.

8. Procédé suivant l'une quelconque des revendications 1 à 7, dans lequel l'hydroxyalkyl sulfonate d'ammonium utilisé dans la partie (c) ne contient pas plus de 0,85% en poids d'alkylène glycol.

9. Procédé suivant l'une quelconque des revendications 1 à 8, dans lequel l'alcanoyloxy alkyl sulfonate d'ammonium a une activité anionique d'au moins 85%.

10. Procédé suivant l'une quelconque des revendications 1 à 9, dans lequel le pH du mélange réactionnel est maintenu en dessous d'environ 7 au cours de la réaction de la partie (a) en (i) établissant le pH initial du mélange réactionnel de bisulfite d'ammonium dans la gamme de 4,5 à 5,2 ou (ii) ajoutant un agent neutralisant acide au mélange réactionnel en fonction des nécessités pour maintenir le pH dans la gamme de 5,5 à 7.

11. Procédé suivant la revendication 10, dans lequel l'acide gras est de l'acide gras de noix de coco.

12. Procédé l'une quelconque des revendications à 11, dans lequel l'alcanoyloxy alkyl sulfonate d'ammonium a une activité anionique supérieure à 85% et dans lequel une solution aqueuse de 25 à 35% en poids de cet alcanoyloxy alkyl sulfonate d'ammonium n'est pas trouble à la température ambiante.

13. Procédé suivant l'une quelconque des revendications 1 à 12, dans lequel l'hydroxyalkyl sulfonate d'ammonium utilisé dans la partie (c) contient moins de 0,5% en poids d'éthylène glycol.

14. Procédé suivant l'une quelconque des revendications 1 à 13, dans lequel l'hydroxyalkyl sulfonate d'ammonium utilisé dans la partie (c) contient moins de 1% en poids de sulfate d'ammonium.

15. Composition détergente liquide claire comprenant une solution aqueuse de l'alcanoyloxy alkyl sulfonate d'ammonium suivant l'une quelconque des revendications 1 à 14.

16. Composition détergente liquide claire suivant la revendication 15, dans laquelle l'alcanoyloxy alkyl sulfonate d'ammonium est du cocoyl iséthionate d'ammonium.
